(19) Europäisches Patentamt
European Patent Office
Office européen des brevets

(11) Veröffentlichungsnummer: **0 622 633 A2**

(12) **EUROPÄISCHE PATENTANMELDUNG**

(21) Anmeldenummer: **94106270.5**

(22) Anmeldetag: **22.04.94**

(51) Int. Cl.5: **G01N 33/94**, //G01N33/535

(30) Priorität: **29.04.93 DE 4314091**

(43) Veröffentlichungstag der Anmeldung:
**02.11.94 Patentblatt 94/44**

(84) Benannte Vertragsstaaten:
**DE ES FR GB IT**

(71) Anmelder: **BOEHRINGER MANNHEIM GMBH**

**D-68298 Mannheim (DE)**

(72) Erfinder: **Hoess, Eva, Dr.**
**Am Mühlberg 1A**
**D-82319 Starnberg (DE)**
Erfinder: **Huber, Erasmus, Dr.**
**St. Willibald 10**
**D-86923 Finning (DE)**
Erfinder: **Markert-Hahn, Christine, Dr.**
**Herzogstandstrasse 2A**
**D-82402 Seeshaupt (DE)**
Erfinder: **Rollinger, Wolfgang, Dr.**
**Kaiser-Heinrich-Strasse 10**
**D-82398 Polling (DE)**

(54) **Immunologisches Nachweisverfahren für Triazine.**

(57) Ein immunologisches Verfahren zur Bestimmung von Triazin und Triazinderivaten durch einen kompetitiven Immunoassay, bei dem Probe und markiertes Atrazin um einen vor, während oder nach der immunologischen Reaktion festphasengebundenen polyklonalen Antikörper konkurrieren, Bindung des immunologischen Komplexes an die Festphase, Trennung von Festphase und ungebundenem markierten Atrazin und Bestimmung der Markierung in der festen und flüssigen Phase als Maß für den Gehalt an Triazin und Triazinderivaten ist dadurch gekennzeichnet, daß polyklonale Antikörper, die durch Immunisierung mit einem 4-Alkylamino-S-Triazin erhalten wurden und als markiertes Atrazin ein Konjugat aus einer Markierung und 4-Amino-S-Triazin, welches über die 6-Position an die Markierung gebunden ist, verwendet werden. Mit diesem Verfahren können Triazin und Triazinderivate gleichzeitig bestimmt werden.

EP 0 622 633 A2

Die Erfindung betrifft ein immunologisches Verfahren zum Nachweis von Triazinen sowie die hierfür verwendeten Reagentien.

Triazine und Triazinderivate, wie Atrazin, Simazin, Ametryn, Propazin, Terbutylazin, Cyanazin, Desethylterbutylazin, sind Substanzen, die als Herbizide in großem Umfang im Pflanzenschutz eingesetzt werden. Da Triazine nur sehr langsam im Boden abgebaut werden und damit ins Grundwasser übertreten können, ist ein schneller und empfindlicher Nachweis für Triazine von besonderer Bedeutung.

Die Bestimmung von Triazinen erfolgt entweder durch instrumentelle Analytik (HPLC- oder GC-MS-Methoden, U. Obst, GIT Fachz. Lab. 4 (1992) 365), mikrobiologisch (R. Reupert, GIT Spezialchromatographie 1 (1992, 30) oder durch immunologische Nachweisverfahren (Achr. Chem. Tech. Lab. 39 (1991) M1 - M42).

Die immunologischen Nachweisverfahren für Triazine werden vorzugsweise als ELISA-Test durchgeführt. Üblicherweise handelt es sich dabei um einen kompetitiven Test, bei dem ein Antikörper gegen ein Triazin oder Triazinderivat immobilisiert wird und das Triazin aus der Probe mit einem Konjugat aus Triazin und einer Markierung, vorzugsweise einem Enzym, kompetiert. Je mehr Triazin in der Probe enthalten ist, um so weniger des enzymmarkierten Konjugats wird an den Antikörper gebunden. Nach Bildung der immunologischen Komplexe wird die Menge des Markierungsenzyms als Maß für die Menge des in der Probe enthaltenen Triazins, üblicherweise photometrisch, bestimmt.

Mit derartigen Immunoassays lassen sich entweder einzelne Triazine bestimmen oder ganze Gruppen von Triazinen. Üblicherweise werden bei den bekannten Immunoassays monoklonale Antikörper (für Tests auf einzelne Triazine) oder polyklonale Antikörper (für Tests auf einzelne Triazine oder Triazingruppen) verwendet sowie ein Konjugat aus einem Enzym, vorzugsweise Peroxidase, und dem Hapten, welches auch bei der Immunisierung verwendet wurde (homologes Konjugat). Ein homologes Enzymkonjugat ist also ein Konjugat aus Hapten und Enzym, bei dem das Hapten im Konjugat und Immunogen bzgl. Substituentenmuster als auch Kupplungsposition des aktivierten Haptens identisch ist.

Mit den bekannten gruppenspezifischen Triazintesten ist es zwar möglich, Atrazin mit einer hohen Empfindlichkeit nachzuweisen, die Empfindlichkeit gegenüber anderen Triazinen ist jedoch gering (L. Weil, Nachr. Chem. Tech. Lab. 39 (1991) 1277).

Aus der EP-A 0 300 381 ist ein immunologisches Nachweisverfahren für Triazine bekannt, bei dem polyklonale Antikörper aus Kaninchen sowie ein Enzymkonjugat aus alkalischer Phosphatase und einem Triazin, das je nach Art des Antikörpers ausgewählt ist, verwendet werden. Wird der Nachweis mit einem Antikörper durchgeführt, der durch Immunisierung mit Ametryn hergestellt wurde, so wird Ametryn im Konjugat verwendet.

Aus der EP-B 0 180 305 ist ein Verfahren zur Bestimmung von Atrazinen bekannt, bei dem ebenfalls zur Immunisierung das gleiche Hapten wie im Enzymkonjugat verwendet wird. Analoge Tests sind beschrieben von B. Dunbar, J. Agric. Food Chem. 38 (1990) 433 - 437, M. H. Goodrow, J. Agric. Food Chem. 38 (1990) 990 - 996. Dort wird zusätzlich ausgeführt, daß derartige homologe Konjugate (bei denen die Bindung des Triazins an das Trägerprotein für die Immunisierung und an das Enzym im Konjugat über die gleiche Stelle erfolgt) bessere Ergebnisse im Test ergeben, wie wenn ein heterologes Konjugat (bei dem die Bindung von immunologischem Träger und Enzym über unterschiedliche Positionen am Triazinring erfolgt) verwendet wird.

Mit den bekannten Tests wird jedoch für einige Triazine nur eine geringe Empfindlichkeit erreicht, so daß nicht festgestellt werden kann, ob die Verunreinigung der Probe mit Triazinen insgesamt unter dem vom Gesetzgeber festgelegten Wert liegt. Eine vollständige Erfassung aller Triazinderivate in Wasserproben ist demnach durch die bekannten immunologischen Tests nicht gewährleistet.

Aufgabe der vorliegenden Erfindung ist es deshalb, ein Verfahren zur Bestimmung von Triazin und Triazinderivaten zur Verfügung zu stellen, mit dessen Hilfe ein Screening von triazinverunreinigten Wasserproben möglich ist, eine hohe Sensitivität und Kreuzreaktivität zu Atrazin, Ametryn, Propazin, Terbutylazin, Simazin, Cyanazin und Desetylterbutylazin erreicht wird und ein Nachweis der Mengen in den gesetzlichen Grenzwerten von 0,1 $\mu$g/l Atrazin und 0,5 $\mu$g/l Gemisch aus Triazinen möglich ist.

Die Aufgabe der vorliegenden Erfindung wird gelöst durch ein immunologisches Verfahren zur Bestimmung von Triazin und Triazinderivaten durch einen kompetitiven Immunoassay, bei dem Probe und markiertes Atrazin um einen vor, während oder nach der immunologischen Reaktion festphasengebundenen polyklonalen Antikörper konkurrieren, Bindung des immunologischen Komplexes an die Festphase, Trennung von Festphase und ungebundenem markiertem Atrazin und Bestimmung der Markierung in der festen oder flüssigen Phase, als Maß für den Gehalt an Triazin und Triazinderivaten, dadurch gekennzeichnet, daß polyklonale Antikörper, die durch Immunisierung mit einem 4-Alkyl-amino-S-Triazin erhalten wurden, und als markiertes Atrazin ein Konjugat aus einer Markierung und 4-Amino-S-Triazin, welches über die 6-Position an die Markierung gebunden ist, verwendet werden.

Es hat sich überraschenderweise gezeigt, daß bei Kombination von derartig hergestellten polyklonalen Antikörpern und Konjugaten eine hohe Kreuzreaktivität des Bestimmungsverfahrens für Atrazin, Terbutylazin, Desethylterbutylazin, Propazin, Simazin, Cyanazin, Desethylatrazin und Desisopropylatrazin erreicht wird. Auf diese Weise kann auch der Gehalt an Atrazin und Atrazinderivaten bestimmt werden. Die Kreuzreaktivität bei Verwendung eines homologen Konjugats (Hapten in Immunogen und Konjugat identisch) zeigt dagegen eine wesentlich schlechtere Kreuzreaktivität.

Als Immunogene werden Triazine verwendet, welche in 4-Position einen Alkylaminorest tragen und über die 6-Position an den immunogenen Träger gebunden sind. Als Alkylreste werden kurzkettige, verzweigte und unverzweigte Alkylreste mit 1 - 4 C-Atomen verwendet. Besonders bevorzugt wird der Isopropylrest verwendet.

Die Kopplung an den immunogenen Träger (z. B. KLH, $\beta$-Galactosidase, Edestin) erfolgt vorzugsweise durch nucleophile Substitution. Dazu wird eine substituierte Aminogruppe eingeführt, deren Substituent zur Kopplung an den immunologischen Träger geeignet ist. Beispielsweise wird als aktivierte Gruppe eine Carboxylgruppe eingeführt, die zum Hydroxysuccinimid aktiviert wird und anschließend über die Aminogruppe des immunologischen Trägers gekoppelt wird. Weitere Kopplungsmethoden sind dem Fachmann geläufig (M. Brinkley, Bioconjugate Chem. 3 (1992) 2 - 13).

Die Herstellung des polyklonalen Antiserums erfolgt ebenfalls nach den üblichen, dem Fachmann geläufigen Methoden, vorzugsweise in Schafen. Eine derartige Immunisierung ist beispielsweise bei B. Dunbar, J. Agric. Food Chem. 38 (1990) 433 - 437 und N.H. Goodrow, J. Agric. Food Chem. 38 (1990) 990 - 996 beschrieben. Die erhaltenen polyklonalen Antiseren können ohne weitere Reinigung eingesetzt werden.

Im Konjugat aus Markierung und Triazinderivat kann als Markierung jede dem Fachmann geläufige Markierung verwendet werden. Bevorzugt wird jedoch eine Enzymmarkierung (Peroxidase, alkalische Phosphatase, $\beta$-Galactosidase) verwendet. Die Kopplung des Triazinderivats an das Enzym erfolgt über 6-Position. Zur Herstellung dieser Konjugate wird in 6-Position zunächst über die Aminogruppe eines heterobifunktionellen Spacers Chlornucleophil substituiert. Als zweite Funktion des Spacers kann jede aktivierbare Gruppe verwendet werden. Vorzugsweise wird jedoch eine Thiolgruppe, die S-Acetyl-geschützt ist, verwendet. Die Länge des Spacers zwischen Triazin und Peroxidase ist unkritisch, beträgt jedoch zweckmäßig zwischen 10 und 30 Atomen. In einem weiteren Reaktionsschritt wird dann das Chlor in 4-Position durch eine Aminogruppe substituiert. Anschließend wird die geschützte Thiolfunktion freigesetzt und zweckmäßig über ein Maleinimido-funktionalisiertes Enzym Triazin und Enzym gekoppelt.

Die Durchführung der immunologischen Bestimmung von Triazin und Triazinderivaten erfolgt nach den dem Fachmann geläufigen Verfahren. Derartige Verfahren sind beispielsweise in S. Wüst, Git Fachz. Labor 2 (1990) 99, S.J. Huber, Git Fachzt. Labor 10 (1985) 969, B. Dunbor, JU. Agric. Food. Che. 38 (1990) 433, M.H. Goodrow, J. Agric. Food. Chem. 38 (1990) 990 beschrieben. Üblicherweise wird hierzu das polyklonale Antiserum an einen Träger, wie Mikrotiterplatten, gebunden. Diese Bindung kann adsorptiv, kovalent oder über ein weiteres Bindungspaar, wie beispielsweise

Beispiel 1

Herstellung des Triazin-Immunogens

Beispiel 1.1

2,4-Dichlor-6-tert.butylamino-1,3,5-triazin

9.23 g (50 mmol) Cyanurchlorid in 200 ml Toluol gelöst werden mit 10 ml 0.1 n Natronlauge und 6.74 g (92.25 mmol) tert. Butylamin versetzt und 1 h bei 20 °C gerührt. Der pH-Wert der Reaktion wird in dieser Zeit ständig kontrolliert und ggf. mit 1 n Natronlauge auf pH 11-12 nachgestellt. Anschließend wird das Lösungsmittel abdestilliert, der Rückstand in 150 ml Wasser aufgenommen, zweimal mit je 150 ml Essigester extrahiert, die organische Phase über Magnesiumsulfat getrocknet und das Lösungsmittel entfernt. Der Rückstand wird in 200 ml Essigester/Petrolether (v/v 4/1) gelöst, unlösliche Bestandteile abfiltriert und das Filtrat eingeengt. Die Reinigung des Rohprodukts erfolgt durch Säulenchromatographie-(Kieselgel Merck 60, 5 x 30 cm, Eluent: Essigester/Petrolether (v/v 4/1)).

| Ausbeute: | 8.84 g (74 % d. Th.) |
| --- | --- |
| $^1$H-NMR (D$_6$-DMSO/TMS): | 1.36 (s, 9H, CH$_3$); 8.91 ppm (s, 1H, NH). |
| DC: | Kieselgel (Merck 60), Essigester/Petrolether (v/v 4/1). |
| | $R_f = 0.88$ |

Beispiel 1.2

6-[N-(6-Aminohexylcarboxyl)]-2-chlor-4-tert.butylamino-1,3,5-triazin

Zu einer Lösung aus 4.42 g (20 mmol) der Verbindung aus Beispiel 1.1 in 100 ml Toluol werden langsam 5.25 g (40 mmol) 6-Aminocapronsäure in 20 ml 0.1 n Natronlauge getropft. Der pH-Wert wird mit 1 n Natronlauge auf pH 11-12 nachgestellt und das Reaktionsgemisch 3 h unter Rückfluß gehalten. Anschließend wird das Lösungsmittel im Wasserstrahlvakuum entfernt, der Rückstand in wenig THF gelöst und langsam zu 500 ml eisgekühltem Diisopropylether getropft. Die Suspension wird noch 30 min unter Eiskühlung gerührt, der Niederschlag abgesaugt und im Hochvakuum getrocknet.

Ausbeute: 0.76 g (6 % d. Th.)

$^1$H-NMR (D$_6$-DMSO/TMS): 1.34 (s, 9H, C$\underline{H}_3$), 1.48 (m, 6H, C$\underline{H}_2$); 2.12 (t, 2H, J = 7.0 Hz, C$\underline{H}_2$-COOH); 3.17 (m, 2H, C$\underline{H}_2$-NH); 7.39 (s br, 1H, N$\underline{H}$-C); 7.75 ppm (m, 1H N$\underline{H}$-CH$_2$); COOH tauscht unter der Aufnahmebedingungen aus.

DC: Kieselgel (Merck 60 ), Essigester/Methanol (v/v 3/1) und 1% Essigsäure. R$_f$ = 0.77

Beispiel 1.3

Methansulfonsäure-N-hydroxysuccinimidester

1.15 g (10 mmol) N-Hydroxysuccinimid werden in 10 ml wasserfreiem THF gelöst und langsam mit 1.6 ml Triethylamin versetzt. Die entstandene Suspension wird mit einer Eis/Kochsalzmischung gekühlt und bei 0-5°C unter Rühren mit 0.78 ml (10 mmol) Methansulfonylchlorid versetzt. 30 min wird bei 0°C nachgerührt, danach läßt man die Temperatur auf 20°C ansteigen. Die Mischung wird über Nacht bei 20°C stehen gelassen und anschließend im Vakuum eingedampft. Der kristallisierte Rückstand wird in Wasser aufgenommen, abgesaugt und gut mit Wasser gewaschen. Im Exsiccator wird das Produkt über Sicapent bis zur Gewichtskonstanz getrocknet.

Ausbeute: 1.63 g (84 % d. Th.)

Beispiel 1.4

Aktiviertes Triazin-Hapten

633 mg (2 mmol) der Verbindung aus Beispiel 1.2 werden in 30 ml Methanol suspendiert und mit 113.4 mg (1.6 mmol) festen Kaliumhydroxid versetzt. Die sich bildende klare Lösung wird 20 min bei 20 °C gerührt und anschließend das Lösungsmittel im Wasserstrahlvakuum abdestilliert. Der Rückstand wird in 20 ml THF aufgenommen, mit 386.3 mg (2 mmol) Methansulfonsäure-N-hydroxysuccinimidester aus Beispiel 1.3 und katalytischen Mengen Dibenzo-18-krone-6 versetzt und 21 h bei 20 °C gerührt. Das Lösungsmittel wird abdestilliert, der Rückstand in 50 ml Essigester aufgenommen und Unlösliches abfiltriert. Das Filtrat wird einmal mit 50 ml ges. Natriumhydrogencarbonat-Lösung und zweimal mit Wasser extrahiert, über Magnesiumsulfat getrocknet und am Hochvakuum eingeengt.

Ausbeute: 710 mg (86 % d. Th.)

$^1$H-NMR (D$_6$-DMSO/TMS): 1.37 (s, 9H, C$\underline{H}_3$); 1.40 (m, 2H, C$\underline{H}_2$); 1.53 (m, 2H, C$\underline{H}_2$); 1.64 (m, 2H, C$\underline{H}_2$); 2.65 (t, 2H, J = 7.0 Hz, C$\underline{H}_2$-COO); 2.82 (s, 4H, C$\underline{H}_2$COO); 3.33 (m, 2H, C$\underline{H}_2$-NH); 7.45 (s, 1H, N$\underline{H}$-C); 7.80 ppm (t, 1H, J = 6.8 Hz, N$\underline{H}$-CH$_2$).

$^{13}$C-NMR (D$_6$-DMSO/TMS): 23.88, 25.23 ($\underline{C}$H$_2$); 25.35 ($\underline{C}$H$_3$); 28.10, 30.08 ($\underline{C}$H$_2$); 39.40 ($\underline{C}$H$_2$-NH); 50.50 ($\underline{C}$-(CH$_3$)$_3$); 164.56, 164.89 (N = $\underline{C}$-N); 167.23 ($\underline{C}$-Cl); 168.74 ($\underline{C}$OO); 170.07 ppm (O$\underline{C}$O-CH$_2$).

MS (EI): 412 [M]$^+$

DC: Kieselgel (Merck 60), Essigester/Methanol (v/v 3/1). R$_f$ = 0.91

Beispiel 1.5

Triazin-Immunogen

Zu einer Lösung aus 4 g (2.1 x $10^{-6}$ mol, 1 g $\beta$-Gal-Wirkstoff) $\beta$-Galactosidase-Lyophilisat in 100 ml Wasser, mit 0.1 n Natronlauge auf pH 8.5-9.0 eingestellt, tropft man 157 mg (3.8 x $10^{-4}$ mol) der Verbindung aus Beispiel 10 in 20 ml Dioxan gelöst. Nach 30 min wird der pH-Wert auf pH 8.5-9.0 nachgestellt und der Ansatz weitere 16 h bei 20°C gerührt. Anschließend wird die Reaktionslösung in einer Amiconzelle aufkonzentriert, mit 50 ml 10 mmol $KPO_4$-Puffer pH 7.0, der 0.9 % Natriumchlorid enthält, versetzt. Das Immunogen wird über eine AcA 202-Gelsäule (3 x 50 cm, Eluent: 10 mmol $KPO_4$-Puffer pH 7.0 enthält 0.9 % Natriumchlorid) aufgereinigt. Die Fraktionen des ersten Peaks (UV-Detektor, 280 nm) werden gepoolt.

Beispiel 2

Herstellung des homologen POD-Konjugats

Meerrettich Peroxidase (PODp)[1] wird in 10 mM Kaliumphosphatpuffer pH 8.5, 0.1 M NaCl, 0.25 mM $CaCl_2$ und das aktivierte Triazin-Hapten aus 1.3 in DMSO gelöst. Die entsprechenden Mengen PODp und Triazin werden gemischt, so daß eine molare Stöchiometrie von 5 : 1 (Hapten:Protein) resultiert. Die DMSO-Konzentration beträgt bei der Kopplung 20 %. Die Inkubation erfolgt 2 h bei 25°C unter Rühren. Anschließend wird zur Abtrennung des nicht abreagierten Haptens über Nacht dialysiert gegen 10 mM Kaliumphosphatpuffer, pH 7.0, 0.1 M NaCl, 0.025 mM $CaCl_2$.

Beispiel 3

Herstellung des heterologen Triazin-Konjugats

Beispiel 3.1

1-Butoxycarbonyl-1,2-diaminoethan (m-Boc-ED)
Zwischenprodukt

Zu einer Lösung aus 120 g (2 mol) Ethylendiamin und 2 l Dioxan/Wasser (v/v 1/1) werden unter Rühren bei 0°C 218 g (1 mol) Di-tert.butyldiarbonat in 1 l Dioxan innerhalb 1 h zugetropft. Nach einstündigem Rühren bei 20°C engt man das Lösungsmittel im Wasserstrahlvakuum ein, filtriert vom als Nebenprodukt entstandenen Bis-1,2-butoxycarbonyl-1,2-diaminoethan ab und extrahiert das Filtrat mit 300 ml Essigester. Die organische Phase wird noch dreimal mit je 100 ml Wasser gewaschen, über Natriumsulfat getrocknet und im Wasserstrahlvakuum eingeengt.

| | |
|---|---|
| Ausbeute: | 27.3 g (8.5 % d. Th.) |
| $^1$H-NMR (CDCl$_3$/TMS): | 1.45 (s, 9H, O-C(C$\underline{H}_3$)$_3$); 2.79 (T, 2H, J = 7.5 Hz, C$\underline{H}_2$-NH); 3.14 (m, 4H, CON$\underline{H}$) 5.00 ppm (s, br, 1H, O-C$\underline{H}_2$-NH$_2$); |
| DC: | Kieselgel 60 (Merck), Isopropanol/Butylacetat/Wasser/Ammoniak (v/v/v/v/ 50/30/15/5), Detektion mit Ninhydrin. |
| | R$_f$ = 0.23 |

Beispiel 3.2

1-Butoxycarbonyl-2-[(3-methylcarbonylthio)propionyl]-1,2-diaminoethan (Boc-ED-(S)ATP)

8.00 g (50 mmol) Verbindung aus Beispiel 3.1 und 12.26 g (50 mmol) N-Succinimidyl-S-acetylthiopropionat (SATP) werden in 100 ml Tetrahydrofuran gelöst und 4 h bei 20°C gerührt. Anschließend kühlt man die Lösung im Eisbad, saugt das ausgefallene Produkt ab und trocknet es im Hochvakuum bei 40°C.
Ausbeute: 9.22 g (63.9 % d. Th.)

[1] Vorpolymerisierte POD (PODp) kann durch Reaktion von monomerer POD mit Glutardialdehyd, wie bei Engvall und Perlmann beschrieben (Immunochemistry 8 (1971), 871 - 874), erhalten werden.

## Beispiel 3.3

### 1-[(3-Methylcarbonylthio)propionyl]-1,2-diaminoethan-Trifluoracetat (ED-(S)ATP x TFA)

7.25 g (25 mmol) Verbindung aus Beispiel 3.2 werden in 10 ml Trifluoressigsäure gelöst und 4 h bei 20°C gerührt. Anschließend wird die Lösung im Wasserstrahlvakuum zur Trockne eingedampft und der Rückstand mit 50 ml Diisopropylether digeriert. Das Produkt wird im Hochvakuum getrocknet und kristallisiert nach einer längeren Induktionsphase aus.

Ausbeute: 5.54 g (65 % d. Th.)

$^1$H-NMR ($D_6$-DMSO/TMS): 2.31 (S, 3H, -C$\underline{H}_3$); 2.39 (t, 2H, J = 7.0 Hz, C$\underline{H}_2$-CO); 2.85 (t, 2H, J = 7.0 Hz, C$\underline{H}_2$S); 3.00 (t, 2H, J = 7.0 Hz, C$\underline{H}_2$NH); 3.26 (t,2H, J = 7.0 Hz, C$\underline{H}_2$NH); 7.92 (s, br, N$\underline{H}$); 8.13 ppm (s, br, 1H, N$\underline{H}$-CO)

DC: Kieselgel 60 (Merck), Essigester/Eisessig/Wasser (v/v/v 5/5/2), Detektion mit Ninhydrin.

$R_f$ = 0.62

## Beispiel 3.4

### 6-[N-[3-Methylcarbonylthio)-propionyl]-1,2-diaminoethyl]-2,4-dichlor-1,3,5-triazin (Dichlortriazin-ED-(S)ATP)

1.52 g (5 mmol) 1-[(3-Methylcarbonylthio)propionyl]-1,2-diamino-ethan-Trifluoracetat-Salz (ED-(S)-ATPXTFA) aus Beispiel 3.3 werden in 50 ml Tetrahydrofuran/Dioxan (v/v 1/4) gelöst und mit 0.51 mg (0.70 ml,5 mmol) Triethylamin versetzt. Anschließend kühlt man das Reaktionsgemisch auf 0 °C und gibt 0.92 g (5 mmol) Cyanurchlorid und 0.51 mg (0.70 ml, 5 mmol) Triethylamin zu. Während dem 3-stündigen Rühren unter Eiskühlung kontrolliert man den pH-Wert der Reaktionslösung und stellt ggf. mit Triethylamin auf pH 6 nach. Der Niederschlag wird abgetrennt und das Lösungsmittel ohne Erwärmen im Hochvakuum abdestilliert. Der Rückstand wird in wenig Essigester/ Hexan (v/v 9/1) gelöst und mittels Säulenchromatographie-[Kieselgel 60 (Merck) 4x11 cm, Eluent: Essigester/Hexan (v/v 9/1)] gereinigt.

Ausbeute: 935 mg (55.3 % d. Th.)

$^1$H-NMR ($D_6$-Aceton/TMS): 2.27 (s, 3H, C$\underline{H}_3$); 2.45 (t, 2H, J = 7.0 Hz, C$\underline{H}_2$S); 3.07 (t, 2H, J = 7.0 Hz); 3.53 (m, 4H); 7.30 ppm (s br, 1H, N$\underline{H}$).

$^{13}$C-NMR ($D_6$-Aceton/TMS): 25.4 ($\underline{C}H_2$S); 30.5 ($\underline{C}H_3$); 36.4 ($\underline{C}H_2$CO); 39.1 und 42.4 ($\underline{C}H_2$N); 170.3 und 171.2 (Triazin-Ring); 171.9 ($\underline{C}$ONH); 195.6 ppm ($\underline{C}$SNH).

DC: Kieselgel 60 (Merck), Essigester/Hexan (v/v 9/1).

$R_f$ = 0.57

## Beispiel 3.5

### Aktiviertes Triazin-Hapten

338 mg (1 mmol) der Verbindung aus Beispiel 3.4 werden mit 17 ml einer Dioxan/Ammoniak-Lösung (1 l Dioxan enthält 0.6 m NH$_{3Gas}$, 17 ml = 10 mmol) versetzt. Nach 6-stündigem Rühren bei 20 °C wird das Lösungsmittel im Wasserstrahlvakuum entfernt und der Rückstand mittels präparativer HPLC (Wasser/Acetonitril 0.1 % TFA) gereinigt.

Ausbeute: 126 mg (34 % d. Th.)

$^1$H-NMR ($D_6$-DMSO/TMS): 2.31 (s, 3H, C$\underline{H}_3$); 2.36 (t, 2H, J = 6.9 Hz, C$\underline{H}_2$-S); 2.99 (t, 2H, J = 6.9 Hz, C$\underline{H}_2$CO); 3.21 (m, 4H, C$\underline{H}_2$NH); 7.33 (s, br, 2H, N$\underline{H}_2$); 7.66 (t, br, 1H, J = 7.0 Hz, N$\underline{H}$); 8.03 ppm (t, br, 1H, J = 7.0 Hz, N$\underline{H}$).

$^{13}$C-NMR ($D_6$-DMSO/TMS): 24.4 ($\underline{C}H_2$-S); 30.5 ($\underline{C}H_3$CO); 34.8 ($\underline{C}H_2$CO); 38.0, 39.6 ($\underline{C}H_2$NH); 165.5, 166.8, 168.8 (N = $\underline{C}$-N); 170.2 ($\underline{C}$ONH); 195.5 ppm ($\underline{C}$OS).

FAB-MS: 319 [M + H]$^+$

DC: Kieselgel 60 (Merck), Essigester/Methanol (v/v 9/1).

$R_f$ = 0.61

### Beispiel 3.6

### Atra(Deethyl-4-ED)-SATP-PODp(MH)

Zur Aktivierung der PODp wird die PODp mit MHS (Maleimidohexanoyl-N-hydroxy-succinimidester) im molaren Verhältnis von 20 : 1 (Hapten zu Protein) umgesetzt. Das überschüssige MHS wird dann durch Dialyse entfernt. Zur Freisetzung der geschützten SH-Gruppen im Hapten wird eine Hydroxylaminolyse durchgeführt (0.1 M Kaliumphosphatpuffer pH 8.0, 0.1 M Hydroxylamin Endkonzentration bei $25\,^{\circ}$C in 1 h). Für die Kopplung wird die maleinimido-aktivierte PODp in 0.01 M Kaliumphosphatpuffer, pH 6.2, 50 mM NaCl, 1 mM EDTA gelöst und mit der entsprechenden Menge aktiviertem Hapten gemischt, so daß eine molare Stöchiometrie von 1 : 1 resultiert. Die DMSO-Konzentration in allen, aktiviertes Hapten enthaltenden Lösungen sollte 20 % betragen, da sonst das Hapten ausfällt. Der Kopplungsansatz wird 1.5 h bei $25\,^{\circ}$C inkubiert. Anschließend werden die noch freien MH-Gruppen auf der POD mit 1/100 des Volumens an 0.2 M Cysteinlösung abgesättigt (30 min., $25\,^{\circ}$C). Zur Abtrennung der niedermolekularen Bestandteile wird der Kopplungsansatz über Nacht gegen 10 mM Kaliumphosphat-Puffer pH 7.0, 0.1 M NaCl, 0.025 mM $CaCl_2$ dialysiert.

### Beispiel 4

### Immunisierung und Prüfung der Antiseren

10 Schafe werden mit Triazin-Immunogen gemäß Beispiel 1 in Freund'schem Adjuvans immunisiert. Die Dosis beträgt 500 g je Tier. Die Immunisierungen werden über 6 Monate oder länger jeweils in Abständen von 4 Wochen wiederholt. Monatlich einmal werden von allen Tieren Antiserumproben entnommen und auf Anwesenheit von Triazin-Antikörpern untersucht. Zu diesem Zweck wird in einem kompetitiven ELISA mit dem heterologen POD-Konjugat Atra(Deethyl-4-ED)-SATP-PODp(MH) gemäß Beispiel 3 eine Reihe von Terbutylazin-Standards vermessen. Die Durchführung dieser Messung ist in Beispiel 5 ausführlich beschrieben.

Als tauglich werden solche Antiseren ausgewählt, die ausreichend hohes Meßsignal liefern (mind. 1000 mE nach 30 - 60 Min. Farbentwicklung) und mit Terbutylazin gut reagieren
(50 % Verdrängung mit weniger als 300 ng/l Terbutylazin).

### Beispiel 5

### Bestimmung der Konzentration verschiedener Triazine

### Verwendete Lösungen

Beschichtungspuffer: 50 mM Natriumbicarbonat; 0.1 % Natriumazid; pH 9,6
Inkubationspuffer: 10 mM Natriumphosphat; 0,1 % Tween 20 (Fa. Brenntag, Best.Nr. 460761); 0,9 % NaCl; 1 % Crotein C (Fa. CRODA GmbH, Best.Nr. 38241422); pH 7,4
Waschlösung: 0,9 % NaCl; 0,1 % Tween 20;
Substratlösung: Substratlösung Enzymun (Boehringer Mannheim GmbH, Best.Nr. 857424), enthält 1,9 mM ABTS und 3,2 mM Natriumperborat in Phosphat-Citrat-Puffer pH 4,4) mit 2 mg/ml Vanillin

### Durchführung

### Beschichtung:

Mikrotiterplatten (Maxisorp F96, Fa. Nunc, Best.Nr. 4-42404) werden mit Antikörpern gegen den Fc-Teil von Schaf-IgG beschichtet, die durch immunsorptive Reinigung der IgG-Fraktion aus Esel-Antiserum gewonnen worden sind. Der Esel-Antikörper wird in der Konzentration 10 g/ml Protein im Beschichtungspuffer gelöst und in jeden Napf der Mikrotiterplatte 100 $\mu$l dieser Lösung pipettiert. Nach einstündiger Inkubation bei Raumtemperatur unter Schütteln wird die Lösung ausgeschüttet und die Platte dreimal mit Waschlösung gewaschen.

Bindung der Triazin-Antikörper:

Schaf-Antiserum gegen Triazin wird 1 : 50000 mit Inkubationspuffer verdünnt. Jeder Napf der Mikrotiterplatte wird mit 100 μl der verdünnten Lösung beschickt. Während der Inkubationszeit (1 Stunde, Bedingungen wie oben) binden die IgG-Moleküle aus dem Schafserum an die wandgebundenen Esel-Antikörper. Anschließend wird gewaschen wie oben.

Reaktion mit Triazinen und Triazin-POD-Konjugat:

Es wird eine Reihe von Lösungen hergestellt, die Terbutylazin in den Konzentrationen 1000 / 500 / 250 / 125 / 62,5 / 31 / 16 / 8 / 4 / 0 ng/l sowie das erfindungsgemäße Konjugat Atra(Deethyl-4-ED)-SATP-PODp-(MH) in einheitlicher Konzentration 50 mU/ml im Inkubationspuffer enthalten.

Analog werden Verdünnungsreihen anderer Triazine mit den Maximalkonzentrationen 1000 ng/l (Atrazin, Cyanazin und Desethylterbutylazin), 10000 ng/l (Propazin, Simazin, Desethylatrazin) und 100000 ng/l (Desisopropylatrazin) und Verdünnungsstufen 1 : 2 hergestellt, die ebenfalls das genannte Hapten-POD-Konjugat enthalten.

Die wie beschrieben vorbeschichten Näpfe der Mikrotiterplatte werden mit diesen Lösungen (je 100 μl) gefüllt, 1 Stunde inkubiert und anschließend gewaschen wie beschrieben.

Die freien Haptenmoleküle konkurrieren mit dem Hapten-POD-Konjugat um die limitiert vorhandenen haptenspezifischen Antikörper an der Wand der Näpfe. Es wird umso weniger Konjugat gebunden, je mehr freies Hapten in der Lösung vorhanden ist und je besser das Hapten von den Antikörpern erkannt wird.

Substratreaktion:

Alle Näpfe werden mit je 100 μl Substratlösung gefüllt und unter Schütteln inkubiert, bis die Farbentwicklung in den Proben ohne Hapten subjektiv ausreichend hoch erscheint. Dann wird die Extinktion aller Näpfe als Differenzmessung bei den Wellenlängen 405/492 nm bestimmt.

Auswertung:

In der Grafik Meßsignal als Funktion der eingesetzten Haptenkonzentration wird für jede Triazinverbindung die "relative Affinität", d.h. die Haptenkonzentration bestimmt, deren Meßsignal halb so groß wie das Maximalsignal (bei Haptenkonzentration gleich 0) ist. Dabei wird zwischen den Stützstellen der Kurve linear interpoliert. Daraus ergibt sich die Kreuzreaktion der Triazine, bezogen auf Terbutylazin, als Quotient

$$\frac{\text{relative Affinität Tetbutylazin}}{\text{relative Affinität Triazin XY}} \times 100 \%$$

Als "untere Nachweisgrenze" wird analog für jede Triazin-Verbindung die Konzentration festgestellt, bei der das Meßsignal 80 % des Maximalsignals beträgt.

In einem 2. Versuch wird analog die Kreuzreaktion und die untere Nachweisgrenze für jedes der genannten Triazine bei Einsatz des "homologen" Konjugats Atra (Terbutyl-6ah)-OSu-POD bestimmt. In diesem Fall beträgt die POD-Konzentration 30 mU/ml. Die höchsten Haptenkonzentrationen sind für Terbutylazin 10000 ng/l, für alle Triazine 100000 ng/l.

Acht weitere Haptenkonzentrationen werden durch serielle Verdünnung dieser Lösungen im Verhältnis 1 : 2 hergestellt und jeweils ein Leerwert ohne Triazin mitgeführt.

Ergebnisse:

Von 10 mit dem erfindungsgemäßen Immunogen behandelten Schafen reagieren 3 gut mit dem o.g. heterologen POD-Konjugat.

In Tab. 1 sind die Ergebnisse der beiden Versuche mit einem der ausgewählten Antiseren zusammengestellt.

Durch die erfindungsgemäße Kombination von Antikörper und Konjugat wird sowohl die Nachweisempfindlichkeit als auch die Kreuzreaktion entscheidend verbessert.

Tabelle 1

| Kreuzreaktion und Nachweisempfindlichkeit für Antiserum S 4612 | | | | |
|---|---|---|---|---|
| Hapten | Messung mit homologem POD-Konjugat | | Messung mit heterologem POD-Konjugat | |
| | Kreuzreaktion % | untere Nachweisgrenze (ng/l) | Kreuzreaktion (%) | untere Nachweisgrenze (ng/l) |
| Terbutylazin | 100 | 100 | 100 | 12 |
| Atrazin | 4 | 1.600 | 73 | 17 |
| Desethylterbutylazin | 9 | 430 | 102 | 10 |
| Proppazin | 8 | 620 | 26 | 24 |
| Simazin | 0,9 | 4.100 | 10 | 60 |
| Cyanazin | 11 | 310 | 102 | 10 |
| Desethylatrazin | < 0,3 | 9.500 | 7 | 90 |

## Patentansprüche

1. Immunologisches Verfahren zur Bestimmung von Triazin und Triazinderivaten durch einen kompetitiven Immunoassay, bei dem die Probe und markiertes Atrazin um einen vor, während oder nach der immunologischen Reaktion festphasengebundenen polyklonalen Antikörper konkurrieren, Bindung des immunologischen Komplexes an die Festphase, Trennung von Festphase und ungebundenem markierten Atrazin und Bestimmung der Markierung in der festen oder flüssigen Phase als Maß für den Gehalt an Triazin und Triazinderivaten, dadurch gekennzeichnet, daß polyklonale Antikörper, die durch Immunisierung mit einem 4-Alkylamino-S-Triazin erhalten wurden und als markiertes Atrazin ein Konjugat aus einer Markierung und 4-Amino-S-Triazin, welches über die 6-Position an die Markierung gebunden ist, verwendet werden.

2. Verfahren nach Anspruch 1, dadurch gekennzeichnet, daß polyklonale Antikörper verwendet werden, die durch Immunisierung mit einem 4-Alkylamino-S-Triazin, in dem der Alkylrest 1 - 4 C-Atome enthält, verwendet werden.

3. Verfahren nach Anspruch 1 oder 2, dadurch gekennzeichnet, daß polyklonale Antikörper verwendet werden, die durch Immunisierung mit einem 4-Isopropylamino-S-Triazin erhalten wurden.

4. Verfahren nach den Ansprüchen 1 bis 3, dadurch gekennzeichnet, daß als Markierung ein Enzym verwendet wird.